# EUROPEAN PATENT APPLICATION

(11) **EP 3 626 837 A1**
(43) Date of publication of application: **25.03.2020**
(21) Application number: 18306222.3
(22) Date of filing: 20.09.2018
(51) Int. Cl.: C12Q 1/6886

(54) **METHOD FOR TUMOR RECURRENCY PREDICTION**

(71) Applicant: Worldwide Innovative Network, 94800 Villejuif (FR)
(72) Inventor: LAZAR, Vladimir, 94800 Villejuif (FR); RUBIN, Eitan, 70700 Gedera (IL); ROSENBERG, Shai, 9234517 Jerusalem (IL)
(74) Representative: Cabinet Becker et Associés

(57) **Abstract**

The present invention relates a method for predicting the clinical outcome of patient based on newly identified biomarkers including ROCK1 and a method for selecting a subject susceptible to benefit from a treatment with a ROCK1 inhibitor.

## Description

### FIELD OF THE INVENTION

The present invention relates to the field on oncology, especially cancer prognosis and therapy. More particularly, it relates to a method for predicting the clinical outcome of patient and a method for selecting a subject susceptible to benefit from particular treatments.

### BACKGROUND OF THE INVENTION

Lung cancer is the most common malignancy worldwide with a staggering 1.8 million cases diagnosed per year. Non-small-cell lung carcinoma (NSCLC) accounts for about 85% of all lung cancers.

At diagnosis, there is a strong correlation between the NSCLC tumor stage and survival. Patients with stage I tumors at diagnosis are mostly cured by surgery alone, with a 5-year survival rate of 90%. Unfortunately, only 10% of patients with NSCLC are diagnosed at stage I. More than 60% of patients are diagnosed with advanced or metastatic disease (stage IV), and are not curable with current strategies, with 5-years survival rates below 5% (Siegel et al. A. Cancer statistics. CA Cancer J Clin. 64:9-29 (2014); Lung Cancer Fact Sheet. http://www.lung.org/lung-disease/lung-cancer/resources/facts-figures/lung-cancer-fact-sheet.html. 2015).

The therapeutic care of the patients having lung cancer is primarily based on surgery (stage I, and II and IIIA), radiotherapy and chemotherapy which have to be used according to standard protocols. The curative surgery consists in removal of all the tumoral mass. However, this is not always possible to guarantee the absence of any residual tumoral cell after the ablation of the observable part of the tumor, even by experienced surgeons. This is why, when possible, NSCLC are primarily treated by surgical resection, and radiotherapy and/or chemotherapy are increasingly used both pre-operatively (e.g. neoadjuvant chemotherapy) and post-operatively (e.g. adjuvant chemotherapy).

After curative surgery in stage II and IIIA, half of patients suffering from NSCLC relapse, and 5-year survival does not exceed 40%. Most of the patients suffering from NSCLC relapse within the first 12 months after surgery. Unfortunately, there are few biomarkers in clinical practice that reliably predict this outcome.

Tumor biopsy is currently the standard of care for diagnosis and is also used to guide therapeutic decisions for patients with metastatic NSCLC. Despite significant research progress and new promising drugs that include immune-checkpoint inhibitors, the clinical outcome for patients with NSCLC remains poor. Almost all patients with metastases succumb to their disease, mainly due to secondary resistance and clonal selection of resistant cells.

The current unmet needs in NSCLC are early diagnosis and a treatment strategy using to prevent and delay the emergence of cancer recurrence and secondary resistance. To help achieve these goals, the identification and validation of a new generation of biomarkers is required, especially when no prominent driver oncogene alteration is identified.

The identification of high-risk patients in stage I, II and IIIA is another unmet need that needs overcoming to assign appropriate adjuvant therapies. There is a potential utility of several therapeutic drug classes in the adjuvant setting to control tumor cell dissemination and dormancy, reactivation of micrometastases that precedes rapid metastatic outgrowth leading to death. While immunotherapies such as Wnt, SRC and BPM inhibitors hope to provide new inroads, nowadays the standard-of-care adjuvant treatment in NSCLC is platinum-based chemotherapy, which provides an overall survival benefit for only 7% of patients.

Thus, there is still a need to develop a method for predicting cancer recurrence in patient suffering from NSLCL and to identify new target for adjuvant therapy to strengthen the therapeutic arsenal against lung cancer.

### SUMMARY OF THE INVENTION

To overcome these problems, the inventors identifies new biomarkers which can be used to predict tumor recurrence in patient suffering from NSCLC, notably an early cancer recurrence following curative surgery, namely ROCK1, CSTF3 and ZBED6CL. Based on the knowledge of the risk of an early cancer recurrence, they established a new treatment strategy.

The inventor also provides a method for selecting a subject susceptible to benefit from a treatment with a ROCK1 inhibitor, notably in the context of an adjuvant therapy.

The invention relates to the use of ROCK1 as a biomarker for predicting the clinical outcome of a subject having a NSCLC, optionally in combination with CSTF3 and/or ZBED6CL.

The invention also relates to an *in vitro* method for predicting the clinical outcome of a subject suffering from Non-small cell lung cancer (NSCLC), wherein the method comprises determining the expression level of ROCK1 in a tumor sample in comparison to a normal sample from the same patient, the expression level being indicative of the clinical outcome.

Particularly, the high level expression of ROCK1 in the tumor sample in comparison to the normal sample is predictive of a poor prognosis. Preferably, a poor prognosis is a poor or shorter survival, an increased disease recurrence, or an early disease recurrence, even more preferably a high risk of relapse after curative surgery, especially a high risk of early relapse after curative surgery.

In one aspect, the tumor sample and the normal sample are histologically matched.

Particularly, the method further comprises determining the expression level of CSTF3 and/or ZBED6CL in a tumor sample in comparison to a normal sample from the same patient, the high expression level of CST3 and/or ZBED6CL being indicative of a poor prognosis.

In another aspect, the invention relates to a method for selecting a subject susceptible to benefit from a treatment with a ROCK1 inhibitor, wherein the method comprises:
(a) determining the likelihood of tumor recurrence in a subject suffering from a NSCLC by the method according to any one of claims 2-6, and
(b) selecting a subject with a high likelihood of cancer recurrence as susceptible to benefit from a treatment with a ROCK1 inhibitor.

Particularly, the subject had a cancer surgical resection.

Preferably, the expression level of ROCK1 and optionally of CST3 and/or ZBED6CL, is determined by measuring the quantity of the mRNA transcripts, for instance by quantitative RT-PCR, real time quantitative RT-PCR or RNA-seq.

The invention also concerns a ROCK1 inhibitor for use in the treatment of a NSCLC cancer in a subject, the subject having a high level expression of ROCK1 in the tumor sample in comparison to the normal sample, optionally with a high level expression of CST3 and/or ZBED6CL in the tumor sample in comparison to the normal sample.

Preferably, the subject has been selected by the method(s) according to the invention.

Particularly, the ROCK1 inhibitor is to be used in an adjuvant therapy.

The invention finally relates to the use of a kit comprising means for measuring the expression level of ROCK1, and optionally of CST3 and/or ZBED6CL, for (i) predicting the clinical outcome of a subject having a NSCLC (ii) selecting a subject affected with NSCLC with a poor prognosis, in particular to have a cancer relapse, especially an early cancer relapse, and/or (iv) determining whether a subject affected with NSCLC is susceptible to benefit from a treatment with a ROCK1 inhibitor.

### BRIEF DESCRIPTION OF THE DRAWINGS

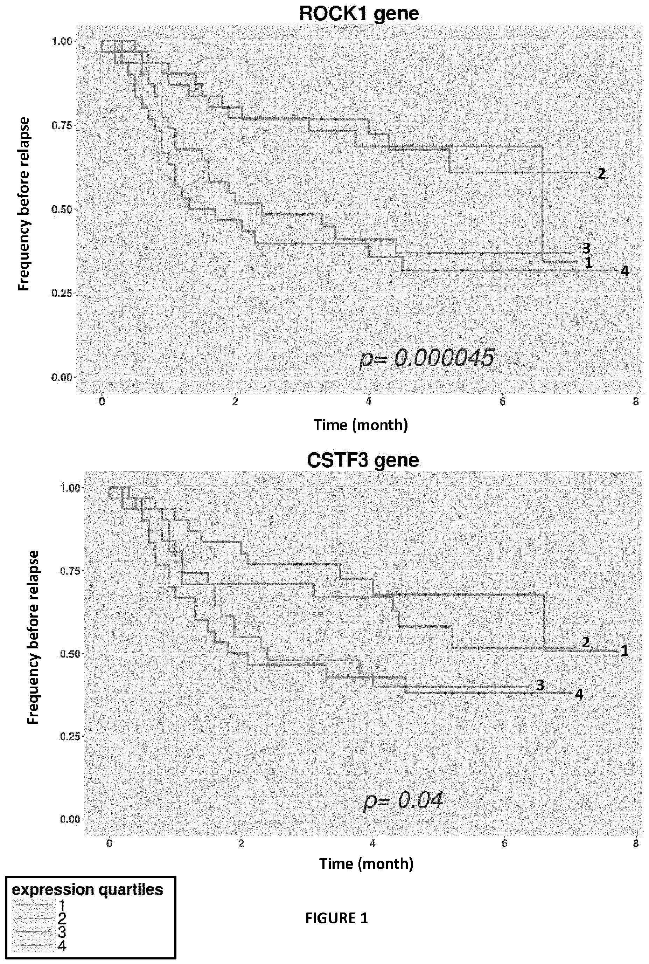

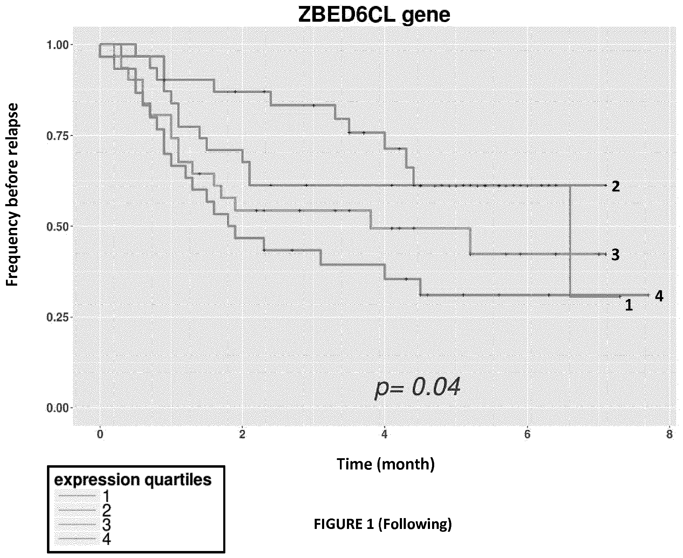

**FIGURE 1**. Kaplan-Meyer curves for the ROCK1, CSTF3, and ZBED6CL genes for which expression levels were significantly associated with survival. For visualization, expression values were discretized into quartiles, and survival curves are shown for each quartile. The P-values in the curves refer to the actual P-values of the cox test and after multiple comparisons adjustment.

### DETAILED DESCRIPTION OF THE INVENTION

### Introduction

The conventional approach to identify new biomarker in cancer generally relies on investigating RNAs only in tumors. This conventional approach did not identify ROCK1 as a potential therapeutic candidate. Surprisingly, the ROCK1 gene was identified as a potential biomarker candidate of clinical outcome through the analysis of its expression in tumoral tissues in comparison to normal tissues from the same patient. Similarly, the genes CSTF3 and ZBED6CL were also identified as potential biomarkers in the same conditions.

The inventors thus provide a method for predicting the clinical outcome of a patient suffering from NSCLC, that comprises determining the expression level of ROCK1 in a tumor sample in comparison to a normal sample from the same patient, the expression level being indicative of the clinical outcome. It is worthwhile to mention that procurement of tumor and normal tissues is very easy during the surgery procedures. Indeed Curative surgery consist in lobectomy (removal of the lobe containing the tumor). The lobe removed is sent to pathologist. it is very easy to dissect tumor and normal tissue distant from the tumor) the High level of expression of ROCK1 alone or in combination with high expression level of CSTF3 and/or ZBED6CL in tumor samples in comparison to their respective expression in normal sample correlates with a poor prognosis, including poor survival prognosis and an early recurrence after curative surgery of NSCLC. Consequently, the inventors consider using ROCK1 as a target for adjuvant therapies after surgical resection of lung cancer in a particular subgroup of patients with high risk of relapse, especially early relapse.

### Definitions

In order that the present invention may be more readily understood, certain terms are defined hereafter. Additional definitions are set forth throughout the detailed description.

Unless otherwise defined, all terms of art, notations and other scientific terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a difference over what is generally understood in the art. The techniques and procedures described or referenced herein are generally well understood and commonly employed using conventional methodologies by those skilled in the art

The term "cancer" or "tumor", as used herein, refers to the presence of cells possessing characteristics typical of cancer-causing cells, such as uncontrolled proliferation, immortality, metastatic potential, rapid growth and proliferation rate, and certain characteristic morphological features. This term includes early stage, localized cancer, later stage, locally advanced cancer; and metastatic stage cancer in any type of patient.

The terms "Non-small-cell lung carcinoma" or "NSCLC", as used herein, is any type of epithelial lung cancer other than small cell lung carcinoma (SCLC). NSCLC includes lung adenocarcinoma, squamous cell lung carcinoma and large-cell lung carcinoma.

The term "cancer sample" or "tumor sample" refers to any sample containing tumor cells derived from a patient or a subject. In particular, tumor cells may be obtained from fluid sample such as blood, plasma, urine and seminal fluid samples as well as from biopsies, organs, tissues or cell samples. In a preferred embodiment, tumor cells are obtained from tumor biopsy or resection sample from the patient. Preferably, the sample contains only tumor cells. Preferably the cancer sample contains nucleic acids and/or proteins. Preferably, cancer samples are NSCLC cancer samples. Preferably, the tumor sample and the normal sample provides from the same type of tissue. More particularly, the tumor and normal samples are histologically matched tissues. The "normal" sample does not comprise any cancer cell. Typically, the samples can be provided by biopsies. In a particular aspect, the tumor sample is a lung cancer adenocarcinoma sample or a sample of derived metastasis and a histologically matched normal sample is a sample from bronchial normal mucosa. Optionally, samples containing tumor cells may be treated prior to their use. As example, a tumor cell enrichment sorting may be performed. The sample may be treated prior to its use. It may be fresh, frozen or fixed (e.g. formaldehyde or paraffin fixed) sample.

As used herein, the terms "subject", "individual" or "patient" are interchangeable and refer to an animal, preferably to a mammal, even more preferably to a human.

As used herein, the terms "marker" and "biomarker" are interchangeable and refer to biological parameters that permit the selection of patients who will have a poor or good prognosis and/or will benefit from a specific treatment. This term refers particularly to "tumor biomarkers". It is a measurable indicator for predicting the clinical outcome of a subject having cancer. Hereafter ROCK1, CST3 and ZEBD6CL are considered as biomarkers.

The term "ROCK1", as use herein, refers to the nucleic acids encoding ROCK1, that is a protein serine/threonine kinase also known as rho-associated, coiled-coil-containing protein kinase 1 that plays a key role in integrin-mediated cell adhesion and migration, in cytoskeleton remodeling and cell motility, in controlling blood-brain barrier and immune cell transmigration, and is involved in TGF-β signaling and β-catenin-dependent Wnt signaling. Preferably, this term refer to human ROCK1. ROCK1 is described in databases under the following accession numbers: Gene ID: 6093, UniGene Hs.306307. This protein is disclosed in UniProt under accession number: Q13464. The GenBank entry of the sequence of the protein and mRNA are respectively: NP_005397.1 and NM_005406.2

The term "CSTF3" as used herein, refers to the nucleic acids encoding Cleavage stimulation factor subunit 3, also known CF-1 77 kDa subunit, Cleavage stimulation factor 77 kDa subunit, CSTF 77 kDa subunit or CstF-77, which is known to be of the multiple factors required for polyadenylation and 3'-end cleavage of mammalian pre-mRNAs. Preferably, this term refer to human CSTF3. CSTF3 is described in databases under the following accession numbers: Gene ID: 1479, UniGene Hs.44402. This protein is disclosed in UniProt under accession number: Q12996. Three isoforms are disclosed and the GenBank entries of the sequence of the protein and mRNA are respectively:
NP_001028677.1, NM_001033505.1
NP_001028678.1, NM_001033506.1
NP_001317.1, NM_001326.2

The term "ZBED6CL" as used herein, refers to the nucleic acids encoding ZBED6 C-terminal-like protein, also known as C7orf29. Preferably, this term refer to human ZB6CL. ZB6CL is described in databases under the following accession numbers: Gene ID: 113763, UniGene Hs.655915. This protein is disclosed in UniProt under accession number: Q96FA7. The GenBank entry of the sequence of the protein and mRNA are respectively: NP_612443.1, NM_138434.2.

The terms "quantity," "amount," and "level" are used interchangeably herein and may refer to an absolute quantification of a molecule in a sample, or to a relative quantification of a molecule in a sample, i.e., relative to another value such as relative to a reference value as taught herein, or to a range of values for the biomarker. These values or ranges can particularly be obtained from a single patient.

As used herein, the "reference value" or "cut-off value" are used interchangeably and refer to a threshold value. It can be an absolute value; a relative value; a value that has an upper and/or lower limit; a range of values; an average value; a median value; a mean value; a statistic value; a cut-off or discriminating value; or a value as compared to a particular control or baseline value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. A reference value can be based on an individual sample value, such as a value obtained from a sample from the individual tested but at an earlier point in time, or a value obtained from a "normal sample" from the same patient.

The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data.

The term "high expression level", "up-regulation" or "overexpression" as used herein, refer to the comparative ratio of the expression of a biomarker (e.g. such as ROCK1) in a tumor sample and in a reference sample, preferably a normal sample. Preferably, a "high expression level" is established when the expression of a biomarker in a tumor sample is at least twice the reference value (e.g. the expression level of the biomarker in the normal sample from the same patient). As used herein, the terms "clinical outcome" and "prognosis" are interchangeable and refer to the determination as to whether a subject is likely to be affected by a cancer relapse, recurrence or metastasis, preferably cancer recurrence, even more preferably a lung cancer recurrence after NSCLC curative surgery, most preferably a NSCLC recurrence after NSCLC curative surgery. The skilled person often makes a prognosis on the basis of one or more diagnosis biomarkers, the presence, absence, or amount of which is indicative of poor prognosis, cancer recurrence or metastasis. These terms also relate to the survival, in particular the overall survival. Overall survival (OS) as used herein refers to the time span from starting the treatment until cancer specific death of the patient.

As used herein, the term "poor prognosis" refers to a decreased patient survival and/or an early disease progression and/or an increased or early disease recurrence. Preferably this term refers to a high risk or likelihood of relapse after NSCLC curative surgery. Reference herein to "cancer recurrence" or "cancer relapse" includes reference to the recurrence of cancer locally, preferably in the lung, or at a remote site or reference to metastasis.

As used herein, the term "ROCK1 inhibitor" can be any compound or treatment capable of inhibiting the expression and/or function of ROCK1, i.e. any compound or treatment that inhibits transcription of the gene, RNA maturation, RNA translation, post-translational modification of the protein and the like.

The term "treatment" refers to any act intended to ameliorate the health status of patients such as therapy, prevention, prophylaxis and retardation of the disease or of the symptoms of the disease. It designates both a curative treatment and/or a prophylactic treatment of a disease. A curative treatment is defined as a treatment resulting in cure or a treatment alleviating, improving and/or eliminating, reducing and/or stabilizing a disease or the symptoms of a disease or the suffering that it causes directly or indirectly. A prophylactic treatment comprises both a treatment resulting in the prevention of a disease and a treatment reducing and/or delaying the progression and/or the incidence of a disease or the risk of its occurrence. In certain embodiments, such a term refers to the improvement or eradication of a disease, a disorder, an infection or symptoms associated with it. In other embodiments, this term refers to minimizing the spread or the worsening of cancers. Treatments according to the present invention do not necessarily imply 100% or complete treatment. Rather, there are varying degrees of treatment of which one of ordinary skill in the art recognizes as having a potential benefit or therapeutic effect.

As used herein the terms "susceptible to", "at high risk for " or "high likelihood of" cancer recurrence or cancer relapse refer to a patient with clinical conditions of developing once more a cancer, preferably a lung cancer, notably shortly after a treatment, preferably after NSCLC surgical resection.

As used herein, the terms "adjuvant therapy", "adjunct therapy" or "add-on therapy" refer to a therapy that is given in addition to a first therapy. Preferably this terms refer to a therapy that is given after the primary or initial therapy to lower the risk of cancer recurrence or relapse. Preferably, such adjuvant therapy is an additional treatment given after NSCLC curative surgery where all detectable disease (e.g. lung tumor cells) has been removed, but where a statistical risk of relapse due to the presence of undetected disease (e.g. lung tumor cells) remains.

### Prognosis

In a first aspect, the invention concerns the use of ROCK1 as a biomarker for predicting the clinical outcome of a subject having a NSCLC. Optionally, ROCK1 can be combined with CSTF3 and/or ZBED6CL, which are both additional biomarker for predicting the clinical outcome. Optionally, the invention further relates to the use of CSTF3 and/or ZBED6CL as a biomarker for predicting the clinical outcome of a subject having a NSCLC.

The clinical outcome is preferably a poor or decreased overall survival, an increased disease recurrence, or an early disease recurrence, even more preferably a high risk of relapse after curative surgery, especially a high risk of early relapse after curative surgery. In particular, the poor or decreased overall survival, the increased disease recurrence, or the early disease recurrence of the subject is with reference to the mean overall survival or disease recurrence or relapse observed in a population of patients suffering from NSLCL or in the total population of NSCLC patients. A poor or decreased overall survival relates to a shorter overall survival. The increased disease recurrence refers to a disease recurrence occurring either earlier when compared with a population of patients suffering from NSLCL or with the total population of NSCLC patients or with a higher intensity, or both. The early disease recurrence or relapse refers to the start of the recurrence or relapse when compared with a population of patients suffering from NSLCL or with the total population of NSCLC patients.

The cancer recurrence, especially the early cancer recurrence, preferably occurs within 1, 2, 3, 4, 5, 6, 7, 8, 9, 10, 11 or 12 months after cancer treatment by any method known by the person skilled in the art, preferably, surgery, radiotherapy, chemotherapy, targeted therapy, immunotherapy or any combinations thereof, even more preferably after curative surgery of the NSCLC cells.

The poor overall survival of a patient is an overall survival decreased by 1, 2, 3, 4, 5, 6, 7, 8, 9, 10, 11 or 12 months in comparison with a total population of patients suffering from NSCLC.

Then, the invention also concerns an *in vitro* method for predicting the clinical outcome of a subject suffering from Non-small cell lung cancer (NSCLC), wherein the method comprises determining the expression level of ROCK1 in a tumor sample in comparison to a normal sample from the same patient, the expression level being indicative of the clinical outcome. The method may comprise determining the expression level of ROCK1 in a tumor sample from the subject, determining the expression level of ROCK1 in a normal sample from the same subject, and comparing the the expression level of ROCK1 in a tumor sample and a normal sample. In another aspect, the invention also concerns an *in vitro* method for predicting the clinical outcome of a subject suffering from Non-small cell lung cancer (NSCLC), wherein the method comprises determining the expression level of CSTF3 and/or ZBED6CL in a tumor sample in comparison to a normal sample from the same patient, the expression level being indicative of the clinical outcome.

In one embodiment, the high level expression of ROCK1 in the tumor sample in comparison to the normal sample is predictive of a poor prognosis. Preferably, a poor prognosis is a poor or decreased overall survival, an increased disease recurrence, or an early disease recurrence, even more preferably a high risk of relapse after curative surgery, especially a high risk of early relapse after curative surgery. Similarly, high level expression of CSTF3 and/or ZBED6CL in the tumor sample in comparison to the normal sample is predictive of a poor prognosis.

The fundamental difference of the present approach from all previous RNA-based studies is the control used. While in most other studies the mRNA levels in each tumor are compared to a common reference, usually obtained by averaging a pool of tumor transcript levels, the present work utilized the normal lung tissue of the same patient as a true reference control.

Thus, the above-mentioned method may also comprise a step of obtaining or providing a sample from the same patient, preferably providing a cancer sample and a normal sample from a patient having NSLCL. In a preferred embodiment, the method is performed on cancer cells from a cancer sample from a patient. The above-mentioned method also necessitates the use of a normal sample, as a way of comparison to the cancer sample. The normal sample is a sample from the same patient, preferably a normal, non-tumor or healthy sample, preferably derived from the same tissue or organ. For example, in the context of NSCLC, the normal sample is a normal lung tissue or sample. Particularly, the normal lung tissue is obtained from lobectomy specimens in patients with resected primary NSCLC tumors. Preferably, the normal and tumor status of tissues used are certified by trained pathologist. To avoid any doubt, surrounding normal tissue in proximity to the tumor is excluded.

The invention also relates to the use of ROCK1, CSTF3 and/or ZBED6CL as biomarkers for predicting the clinical outcome of a subject having a NSCLC.

In one embodiment, the *in vitro* method for predicting the clinical outcome of a subject suffering from Non-small cell lung cancer (NSCLC), comprises determining the expression level of at least one biomarker selected from the group consisting of ROCK1, CSTF3 and/or ZBED6CL in a tumor sample in comparison to a normal sample from the same patient, the expression level being indicative of the clinical outcome. In a preferred embodiment, the biomarker is ROCK1, optionally in combination with CSTF3 and/or ZBED6CL.

The invention also relates to the use of ROCK1 in combination with CSTF3 and/or ZBED6CL as biomarkers for predicting the clinical outcome of a subject having a NSCLC. In this embodiment, the *in vitro* method for predicting the clinical outcome of a subject suffering from Non-small cell lung cancer (NSCLC), comprises:
(a) determining the expression level of ROCK1 in a tumor sample in comparison to a normal sample from the same patient, and
(b) determining the expression level of CST3 and/or ZBED6CL in a tumor sample in comparison to a normal sample from the same patient,
wherein the high expression level of ROCK1 in combination with a high expression level of CST3 and/or ZBED6CL are indicative of a poor prognosis, preferably of a poor survival, an increased disease recurrence, or an early disease recurrence, even more preferably a high risk of relapse after curative surgery, especially a high risk of early relapse after curative surgery.

In particular, the poor prognosis, the poor or decreased overall survival, the increased disease recurrence, or the early disease recurrence of the subject is with reference to the mean overall survival or disease recurrence or relapse observed in a population of patients suffering from NSLCL that do not overexpress ROCK1 and/or CSTF3 and/or ZBED6CL.

### Expression level of ROCK 1, CSTF3 and ZBED6CL

Methods for determining the quantity of mRNA are well known in the art and include, but are not limited to, quantitative or semi-quantitative RT-PCR, real time quantitative or semi-quantitative RT-PCR. Quantitative or semi-quantitative RT-PCR is preferred. Real-time quantitative or semi-quantitative RT-PCR is particularly advantageous. Other methods of Amplification include, but are not limited to, ligase chain reaction (LCR), transcription-mediated amplification (TMA), strand displacement amplification (SDA) and nucleic acid sequence based amplification (NASBA).

Particularly, the quantity of mRNA may be measured using the Nanostring's NCOUNTER™ Digital Gene Expression System (Geiss et al. 2008 Nat. Biotechnol. 26:317-325) which captures and counts individual mRNA transcripts by a molecular bar-coding technology and is commercialized by Nanostring Technologies, or the QuantiGene® Plex 2.0 Assay (Affymetrix). The quantity of mRNA may alternatively be determined using approaches based on high-throughput sequencing technology such as RNA-Seq (Wang et al. Nat Rev Genet. 2009 January; 10(1): 57-63) or sequencing technologies using microfluidic systems or transcriptome approaches. Next Generation Sequencing methods (NGS) may also be used.

The nucleic acid contained in the sample (e.g., cells or tissue prepared from the patient) may be first extracted according to standard methods, for example using lytic enzymes or chemical solutions or extracted by nucleic-acid-binding resins following the manufacturer's instructions. These nucleic acids may be frozen to be stored before use.

In a particular embodiment, the expression level ROCK1, CST3 and/or ZBED6CL is determined by measuring the quantity of the mRNA transcripts, for instance by quantitative RT-PCR, real time quantitative RT-PCR or RNA-seq. Alternatively, the expression level ROCK1, CST3 and/or ZBED6CL is determined by measuring the quantity of protein.

The method comprises the step of determining whether the expression level of ROCK1, CST3 and/or ZBED6CL is dysregulated compared to the reference expression level, e.g. up-regulated or down regulated in comparison to the normal sample, preferably up-regulated compared to the normal sample of the same patient.

Particularly, the differential expression between the expression level of ROCK1, CST3 and/or ZBED6CL in the tumor sample and in the normal sample is expressed in term of "fold change". Particularly, Fold-change is calculated as the ratio of values of the measurement of expression level in the tumor sample and in the normal sample. The expression level of ROCK1, CST3 and/or ZBED6CL in the cancer sample is considered as significantly different (i.e. dysregulated, e.g. up-regulated) compared to the reference expression levels in a normal sample, if, after normalization, differences are in the order of at least 2-fold (or more) higher for the considered biomarker than the expression levels in the normal sample. Preferably, the expression levels of ROCK1, CST3 and/or ZBED6CL in the cancer sample are considered as overexpressed if the expression levels are at least 2-fold higher, or 3, 4, 5 or 6-fold higher for the cancer sample than the respective expression levels in the normal sample from the same patient.

Accordingly "high expression level", "overexpression" or "up-regulation" mean that the biomarker's (e.g. ROCK1, CST3 and/or ZBED6CL) expression level is at least 2, 3, 5 or 6-fold higher in the cancer sample than the expression level in the normal sample from the same patient.

A bivariate analysis can be used to compare gene expression levels in tumor sample vs normal sample: the minimal, maximal and median gene expression levels can be compared and a two sample Wilcoxon rank-sum (Mann-Whitney) test can be used to determine if the minimal, maximal and median gene expression levels are significantly different in the tumor sample compared to the normal sample. The expression level can be studied with a ROC curve analysis, and a cut-off value may then be determined using the Liu method (which defines the optimal cut-point as the point maximizing the product of sensitivity and specificity). This cut-off value may dependent on expression levels of the biomarker(s) in the tumor and normal samples. This cut-off value may be easily adjusted by the skilled person using a particular reference gene. The cut-off value will be chosen so as to obtain a significant p-value.

Typically, the accuracy of the test to discriminate diseased cases from normal sample may be evaluated using Receiver Operating Characteristic (ROC) curve analysis (Metz CE, Semin. Nucl. Med. 1978 Oct; 8(4):283-98; Zweig & Campbell, Clin. Chem. 1993 Apr; 39(4):561-77). In signal detection theory, a ROC curve, is a graphical plot of the sensitivity (or true positive rate), versus false positive rate (1 - specificity or 1 - true negative rate), for a binary classifier system. Each point on the ROC plot represents a sensitivity/specificity pair corresponding to a particular decision threshold. The area under the ROC curve (AUC) is a measure of how well a parameter can distinguish between two diagnostic groups (for example "diseased"/"normal" or "at risk for relapse"/"not at risk for relapse"). In a particular embodiment, the area under the ROC curve (AUC) is a measure of how well a parameter can distinguish between patients with good prognosis and patients with poor prognosis, preferably patients not at risk for NSCLC recurrence and patients at risk for NSCLC recurrence, particularly after NSCLC curative surgery.

For example, the reference value can be expressed as a concentration of the biomarker in the biological sample of the tested subject for a particular specificity and/or sensitivity, or can be a normalized cut-off value expressed as a ratio for a particular specificity and/or sensitivity. If a higher or lower sensitivity and/or specificity is/are desired, the cut-off value can easily be changed by the skilled person in the art, for example using a different reagent for a particular biomarker.

To assess the performance of the models, the AUC (Area Under the Curve) of the receiver operating characteristics curves (ROC) can be computed. The probability of overall survival can be further estimated using the Kaplan Meier method. A log rank test can be performed to test the difference between high- and low-risk groups. High and low-risk prognostic groups were defined according to the cut-off of returned probabilities of 0.5: high risk having a returned probability > 0.5 of death within the first months or first year following the curative surgery of NSCLC cells.

### Subject

The patient is an animal, preferably a mammal, even more preferably a human.

The human patient according to the invention may be a human at the prenatal stage, a newborn, a child, an infant, an adolescent or an adult, in particular an adult of at least 30 years old, preferably an adult of at least 40 years old, still more preferably an adult of at least 50 years old, even more preferably an adult of at least 60 years old.

Preferably, the patient has been diagnosed with a cancer, even more preferably a NSCLC.

In a particular embodiment, the patient has already received at least one line of treatment, by any method known by the skilled person in the art, e.g. surgery, chemo-therapy, radiotherapy or combination thereof, preferably curative surgery for NSCLC.

Preferably, the subject had undergo surgical NSCLC cells resection.

### Subject susceptible to benefit from a ROCK1 inhibitor treatment

The invention also concerns a method for selecting patient susceptible to benefit from a ROCK1 inhibitor treatment, preferably in the context of an adjuvant therapy following a NSCLC curative surgery.

In one embodiment, the invention concerns a method for selecting a subject susceptible to benefit from a treatment with a ROCK1 inhibitor, wherein the method comprises:
(a) determining the clinical outcome in a subject suffering from a NSCLC by any of the methods described hereabove, in particular the overall survival, the disease recurrence or relapse, and
(b) selecting a subject with a poor prognosis, preferably with a high risk of lung cancer recurrence, preferably a high risk of early cancer recurrence, as susceptible benefit from a treatment with a ROCK1 inhibitor.

Accordingly, the present invention relates to a ROCK1 inhibitor for use in the treatment of a NSCLC cancer in a subject, the subject having a high level expression of ROCK1 in the tumor sample in comparison to the normal sample, optionally with a high level expression of CST3 and/or ZBED6CL in the tumor sample in comparison to the normal sample; to the use of a ROCK1 inhibitor for the manufacture of a NSCLC cancer treatment in a subject having a high level expression of ROCK1 in the tumor sample in comparison to the normal sample, optionally with a high level expression of CST3 and/or ZBED6CL in the tumor sample in comparison to the normal sample; and to a method of treating of a NSCLC cancer in a subject, comprising administering a therapeutically effective amount of a ROCK1 inhibitor to the subject having a high level expression of ROCK1 in the tumor sample in comparison to the normal sample, optionally with a high level expression of CST3 and/or ZBED6CL in the tumor sample in comparison to the normal sample.

Preferably, such treatment with a ROCK1 inhibitor is an adjuvant therapy following a curative surgery of NSCLC in a patient.

The results of the cancer sample analysis following the above method allows to discriminate NSCLC patients with poor prognosis, e.g. with higher risk of lung cancer recurrence (e.g. NSCLC) and metastasis within the first months or the first year following curative surgery.

The invention also concerns the use of a ROCK1 inhibitor in an adjuvant therapy following NSCLC curative surgery for a particular subgroup of patients with high risk of relapse within the first months (e.g. up to 12 months) following surgery, e.g. patient in which the tumor sample overexpress ROCK1 compared to a normal sample of the same subject.

The method may further comprises the administration of an effective therapeutic amount of a ROCK1 inhibitor to the patient susceptible to benefit from a ROCK1 inhibitor treatment as selected by the method described herein, preferably shortly after the curative surgery of the lung cancer, even more preferably readily after the NSCLC curative surgery. "An effective therapeutic amount" as used herein refers to the amount of active agent required to confer therapeutic effect on the subject, either alone or in combination with one or more other active agents, e.g. the amount of active agent that is needed to treat the targeted disease or disorder, or to produce the desired effect. The "effective amount" will vary depending on the agent(s), the disease and its severity, the characteristics of the subject to be treated including age, physical condition, size, gender and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment.

Preferably, the ROCK1 inhibitor suitable for the treatment of a patient selected by the method described hereabove is selected from the list consisting of AT13148, GSK429286A GSK180736A (GSK180736), RKI-1447, Y-27632 2 HL, Thiazovivin, Fasydul (HA-1077) HCl, Ripasudil (K-115) hydrochloride dehydrate, Netarsudil (AR-13324) 2HCl, Y-39983 HCl, ZINC00881524, KD025 (SLx-2119), Ripasudil (K-115) hydrochloride dehydrate, Hydroxyfasudil (HA-1100) HCl and XX.

Optionally, the ROCK1 inhibitor can be combined with other active ingredient, in particular with known NSCLC adjuvant therapeutic agent(s) such as platinum-based agent (e.g., vinorelbine, gemcitabine, docetaxel and pemetrexed), an anti-VEGF monoclonal antibody including bevacizumab, or tyrosine kinase inhibitors such as gefitinib, erlotinib, afatinib, and crizotinib.

### Kit and Uses thereof

In another aspect, the invention concerns a kit and its uses, the kit comprising means for measuring the expression level of at least one biomarker selected from the group consisting of ROCK1, CSTF3 and/or ZBED6CL. The kit can be used for (i) predicting the clinical outcome of a subject having a NSCLC (ii) selecting a subject affected with NSCLC with poor prognosis or likely to have a cancer relapse, preferably a NSCLC relapse and/or (iii) determining whether a subject affected with NSCLC, is susceptible to benefit from a treatment with a ROCK1 inhibitor, preferably from an adjuvant therapy comprising a ROCK1 inhibitor following a curative surgery.

In some embodiments, means of taking a sample from an individual and/or of assaying the sample may be provided.

For instance, the means suitable for determining the expression levels of at least one biomarker selected from the group consisting of ROCK1, CSTF3 and/or ZBED6CL can be primers and/or probe specific to ROCK1, CSTF3 and/or ZBED6CL.

Particularly, the kit comprises:
(i) at least one probe specific to mRNA or cDNA of at least one biomarker selected from the group consisting of ROCK1, CSTF3 and ZBED6CL, and, optionally, means for detecting the hybridization of said at least one probe on ROCK1, CSTF3 and ZBED6CL mRNA or cDNA; and/or
(ii) at least one nucleic acid primer pair specific to at least one biomarker selected from the group consisting of ROCK1, CSTF3 and ZBED6CL mRNA or cDNA and, optionally, means for amplifying and/or detecting said mRNA or cDNA; and,
(iii) optionally, a leaflet providing guidelines to use such a kit and method to evaluate the expression level of such biomarkers.

The kits of this invention are in suitable packaging. Suitable packaging includes, but is not limited to, vials, bottles, jars, flexible packaging (*e.g*., sealed Mylar or plastic bags), and the like.

Instructions supplied in the kits of the invention are typically written instructions on a label or package insert (e.g., a paper sheet included in the kit in the form of a leaflet or instruction manual). In some embodiments, the kit can comprise instructions for use in accordance with any of the methods described herein. The kit may particularly comprises a description of the determination of the expression level of at least one biomarker in a tumor sample and in a normal sample and the description of how to proceed to determine whether a biomarker is overexpressed in a tumor sample in comparison to a normal sample from the same patient. The kit may also comprises indication for selecting an individual suitable for a ROCK1 inhibitor treatment based on identifying whether the tumor sample provided from that individual overexpress ROCK1 compared to a normal sample from the same individual, e.g., following methods described herein.

Further aspects and advantages of the present invention will be described in the following examples, which should be regarded as illustrative and not limiting.

### EXAMPLES

The following Figures and Examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### RESULTS

### 1. Analysis of mRNA expression profiles in tumor and normal tissues from NSCLC and ranking of genes expressed differentially in tumor and normal tissue

The inventors analyzed directly the expression in the tumor and the matched normal tissues. To reduce the variability contributed by different tumor types, they focused on one tumor type and evaluated the mRNA expression profiles of the 57 adenocarcinomas. 17,318 genes were ranked by measuring the level of mRNAs in tumors and normal tissues with Agilent microarray technology. The analysis of the 17,318 genes allowed us to identify four main gene-expression profiles in the tumors and their matched normal controls as follow:
***Profile A*** - *Tumor same as Normal:* For 7,556 genes (43%), expression levels were similar in the tumor as compared with matched normal. It is assumed that the expression levels for these genes are driven by the normal tissue and are not a signature of cancer. As a consequence, these genes do not seem to represent promising biomarkers for diagnosis purposes. Nevertheless, it is important to note that profile A shows a striking inter-patient heterogeneity and high dynamic range variability of the expression levels in normal (and tumor) tissues.
***Profile B*** - *Gain in Tumor:* For 4,000 genes (23%), Fold change expression (Fc) was >2 in ≥10% of tumors as compared with matched normal samples and Fc <0.5 in ≤10% of tumors as compared with matched normal samples.
***Profile C*** - *Loss in Tumor:* For 4,195 genes (24%), expression levels in the tumors were lower than in the matched normal samples. Genes were considered as 'Loss in Tumor' if ≥10% of the samples were under-expressed (Fc < 0.5), and ≤10% of the samples were overexpressed (Fc >2).
***Profile D*** - *Mixed differences:* 1,567 genes (9%) represented a mixture of profiles B and C with a threshold ≥10% patients (genes for which FC>2 for >10% of samples and FC<0.5 for >10% of samples). *Assigning genes into change profiles with a different threshold:* If rather than 10% the threshold is set to 50% (expression levels are 2-fold or more in at least 50% of tumor tissues compared to matched normal), then the proportion of genes in each group becomes 91% Profile A (Tumor same as normal), 3% Profile B (Gain in tumor), 4% Profile C (Loss in tumor) and 2% Profile D (Mixed differences).

### 2. Identification of prognostic biomarkers associated with early recurrence after surgery

The expression of all genes were correlated from the four tumor-normal profiles with the clinical outcomes of the patients after surgery. The purpose of this analysis was to identify potential prognostic biomarkers associated with early recurrence after surgery. When analyzing the expression levels normalized by matched normal tissue expression patterns, the inventors identified that high expression levels of the genes *ROCK1* (FDR adjusted p<10⁻⁴), *CSTF3* (FDR adjusted p=0.04) and *ZBED6CL* (FDR adjusted p=0.04) were associated with a shorter time to relapse after surgery (Figure 1 and Table 1. These results were compared with the same analysis using tumor expression alone or analysis of normal tissue expression alone. Neither of these analyses revealed a significant association with time to relapse for any of the genes discovered with the matched-normal-tissues analysis. High expression of ROCK1, CST3 and ZBED6CL was associated with shorter time to relapse after stringent adjustment for multiple comparisons (P<0.05, FDR<0.05).

**Table 1. Genes' significance of associations with time to relapse**

| **Tumor (normal-adjusted)** | |
|---|---|
| **Gene** | **p (adj.)** |
| ROCK1 | 4.6E-05 |
| CSTF3 | 4.0E-02 |
| ZBED6CL | 4.0E-02 |

### METHOD

**Patients and Tissue Samples**: The present *in silico* study used data generated and published by the European Union funded (FP6) Integrated Project CHEMORES initiative (www.chemores.org). Tissue samples from a cohort of 123 patients who underwent complete surgical resection at the Institute Mutualiste Montsouris (IMM, Paris, France) between January 2002 and June 2006 were analyzed. The biobank study was approved by the Ethics Committee. Patient inclusion in the study cohort was determined in a consecutive manner, according to the following criteria: first, availability of tumoral and non-tumoral tissues in the IMM biobank sampled from the surgical resection specimens; second, histological confirmation of NSCLC and non-tumoral tissue; and third, satisfactory RNA quality controls. Patients were followed for up to 4 years after surgery, and the progression-free survival, time to first recurrence, time to death, and survival data were recorded.

**Characteristics of the NSCLC cohort:** The median age of patients was 63 years (range, 41-85 years); 89 patients (72%) were men. The histopathology of all tumors was reviewed by the same pathologist. The most common histologic subtypes of tumor were adenocarcinoma (AC, *n*=57) squamous cell cancer (SCC, *n*=50), large-cell carcinoma (LCC, *n*=13) and others (*n*=3). Using the 7th edition AJCC TNM staging, 16 tumors were stage I, 84 stage II, 15 stage III and 8 stage IV. Adjuvant platinum-based chemotherapy was administered to 61 patients. Fifty-nine patients experienced a relapse. The 2-year relapse-free survival was 64%, and the median time to recurrence for the cohort was 5.2 years. After a median follow up of 40 months (range, 0-92 months, 36 patients died and 23 patients were alive with recurrence.

This study was performed using snap-frozen tumor and matched normal lung tissue, from the same patients following surgical resection of the primary tumor. Macrodissection was performed by trained pathologists, with samples taken from the tumor and from the distant lung normal tissue from the resected lobe. Samples were handled according to the Tumor Analysis Best Practices Working Group. Haematoxylin and eosin stained frozen sections, taken before and after the cutting of slides for molecular analysis, revealed a median cancer cell content in the tumor specimen of 85% (an inter-quartile range of 65-95%). A full description of the genomic investigation is available in. The microarray data related to this study are available from the Array Express Data Repository at the European Bioinformatics Institute.

**Pre-processing**: The input data comprised the dye-swapped expression profiles described in Jyothi Subramanian and Richard Simon, JNCI: Jnl of National Cancer Institute Volume 102, Issue 7 Pp. 464-474. Briefly, two-color analysis was conducted with RNA from the tumor sample compared to the RNA of matched normal dye-swap hybridization of the same samples to a second array. These data were pre-processed minimally by calculating for each gene in each sample a summarized expression value: first, dye swap values were averaged (i.e. green of one array with red of the green of the corresponding array), and second, all the dye-swap averaged intensities of probes for the same genes were averaged.

**Assigning genes into change profiles**: The gene expression in normal and tumor tissue was used to label each sample for every gene, based on its fold-change (Fc) of tumor versus normal tissue, as overexpressed (Fc>2, under-expressed (Fc< 0.5), and the gene was assigned to one of four profiles.

**Median-normalization**: To test the impact of non-matched median normalization, the following procedure was used: (1) samples were randomly divided into two sets: a treatment set and a normalization set; (2) Using a varying set size *k*, the normalization set was further sub-sampled, randomly choosing a subset of k samples; (3) A new normalization value was derived from this set, taking the median of each gene's expression values; and (4) Fold-change was calculated as the ratio of values in the treatment divided by the sub-sample medians from (3). This procedure was performed using normal samples for normal-median normalization and using tumor samples for tumor-median normalization.

**Pathway analysis:** Pathway analysis was carried out using Ingenuity Pathway Analysis (www.qiagen.com/ingenuity). The 500 most frequently upregulated and the 500 most frequently downregulated gene were selected. These genes, and their average-fold change across the cohort, were analyzed for deregulated pathways identification.

**Statistical and Survival Analysis:** Most of the analysis was conducted using Knime (the Konstanz Information Miner), a graphical environment for data analysis pipeline development using R code snippets as required. The complete Knime workflow is available upon request. Survival analysis was carried out on all 123 patients with NSCLC, directly in R, using a univariate Cox test (package survival). Multiple comparison correction was performed on the p-values using False Discovery Rate (FDR) and genes with FDR<0.05 were considered significant. The survival analysis was carried out in three settings of expression profiles: (i) tumor expression levels normalized by matched normal expression; (ii) tumor tissue expression only (iii) normal tissue expression only. It should be noted that survival analysis using tumor expression only is comparable to survival analysis on tumor expression normalized by median expression, because the expression values are divided by a constant value. Ggplot2 package was used for visualization. In order to visualize the association of expression levels and relapse time, expression values were segregated into quartiles and Kaplan-Meyer curves were created with the quartiles as the predictor variable.

**Data Availability**: The data related to this paper have been submitted to the Array Express data repository at the European Bioinformatics Institute (http://www.ebi.ac.uk/arrayexpress/) under the accession numbers E-MTAB-1132. The annotated dataset used in this study is available at: http://erubin85.wixsite.com/website/supporting-material.

## Claims

1. Use of ROCK1 as a biomarker for predicting the clinical outcome of a subject having a NSCLC, optionally in combination with CSTF3 and/or ZBED6CL.

2. An *in vitro* method for predicting the clinical outcome of a subject suffering from Non-small cell lung cancer (NSCLC), wherein the method comprises determining the expression level of ROCK1 in a tumor sample in comparison to a normal sample from the same patient, the expression level being indicative of the clinical outcome.

3. The method according to any one of claim 2, wherein the high level expression of ROCK1 in the tumor sample in comparison to the normal sample is predictive of a poor prognosis.

4. The use of claim 1 or the method according to claims 2 or 3, wherein a poor prognosis is a poor or shorter survival, an increased disease recurrence, or an early disease recurrence, even more preferably a high risk of relapse after curative surgery, especially a high risk of early relapse after curative surgery.

5. The method according to any one of claims 2-4, wherein the tumor sample and the normal sample are histologically matched.

6. The method according to any one of claims 2-5, wherein the method further comprises determining the expression level of CSTF3 and/or ZBED6CL in a tumor sample in comparison to a normal sample from the same patient, the high expression level of CST3 and/or ZBED6CL being indicative of a poor prognosis.

7. A method for selecting a subject susceptible to benefit from a treatment with a ROCK1 inhibitor, wherein the method comprises:
(a) determining the likelihood of tumor recurrence in a subject suffering from a NSCLC by the method according to any one of claims 2-6, and
(b) selecting a subject with a high likelihood of cancer recurrence as susceptible to benefit from a treatment with a ROCK1 inhibitor.

8. The method of any one of claims 2-7, wherein the subject had a cancer surgical resection.

9. The method according to any one of claims 2-8, wherein the expression level of ROCK1 and optionally of CST3 and/or ZBED6CL, is determined by measuring the quantity of the mRNA transcripts, for instance by quantitative RT-PCR, real time quantitative RT-PCR or RNA-seq.

10. A ROCK1 inhibitor for use in the treatment of a NSCLC cancer in a subject, the subject having a high level expression of ROCK1 in the tumor sample in comparison to the normal sample, optionally with a high level expression of CST3 and/or ZBED6CL in the tumor sample in comparison to the normal sample.

11. A ROCK1 inhibitor for use according to claim 10, wherein the subject has been selected by the method according to claim 7.

12. Use of a kit comprising means for measuring the expression level of ROCK1, and optionally of CST3 and/or ZBED6CL, for (i) predicting the clinical outcome of a subject having a NSCLC (ii) selecting a subject affected with NSCLC with a poor prognosis, in particular to have a cancer relapse, especially an early cancer relapse, and/or (iv) determining whether a subject affected with NSCLC is susceptible to benefit from a treatment with a ROCK1 inhibitor.

13. The method according to anyone of claims 7-9, the ROCK1 inhibitor for use according to claim 10 or the use of claim 12, wherein the ROCK1 inhibitor is to be used in an adjuvant therapy.
